(19) Europäisches Patentamt
European Patent Office
Office européen des brevets

(11) EP 1 768 948 B1

(12) **EUROPÄISCHE PATENTSCHRIFT**

(45) Veröffentlichungstag und Bekanntmachung des Hinweises auf die Patenterteilung:
**21.05.2008 Patentblatt 2008/21**

(21) Anmeldenummer: **05758901.2**

(22) Anmeldetag: **12.07.2005**

(51) Int Cl.:
***C07C 211/28*** *(2006.01)* ***C07C 211/29*** *(2006.01)*
***A61K 31/135*** *(2006.01)* ***A61P 13/06*** *(2006.01)*
***A61P 25/22*** *(2006.01)* ***A61P 25/24*** *(2006.01)*
***A61P 29/02*** *(2006.01)*

(86) Internationale Anmeldenummer:
**PCT/EP2005/007537**

(87) Internationale Veröffentlichungsnummer:
**WO 2006/008032 (26.01.2006 Gazette 2006/04)**

(54) **SUBSTITUIERTE AMINOVERBINDUNGEN ALS 5-HT/NA UPTAKEHEMMER**

SUBSTITUTED AMINO COMPOUNDS AS 5-HT/NA UPTAKE INHIBITORS

COMPOSES AMINO SUBSTITUES EN TANT QU'INHIBITEURS DE CAPTAGE DE 5-HT/NA

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.07.2004 DE 102004034619**

(43) Veröffentlichungstag der Anmeldung:
**04.04.2007 Patentblatt 2007/14**

(73) Patentinhaber: **Grünenthal GmbH**
**52078 Aachen (DE)**

(72) Erfinder:
- **BLOMS-FUNKE, Petra**
  **52146 Würselen (DE)**
- **FRIDERICHS, Elmar**
  **52223 Stolberg (DE)**
- **GRAUDUMS, Ivars**
  **52222 Stolberg (DE)**
- **HENNIES, Hagen-Heinrich**
  **52152 Simmerath (DE)**
- **KLESS, Achim**
  **52074 Aachen (DE)**
- **SCHIENE, Klaus**
  **40227 Düsseldorf (DE)**
- **ZIMMER, Oswald**
  **52146 Würselen (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
- **DATABASE CA [Online] CHEMICAL ABSTRACTS SERVICE, COLUMBUS, OHIO, US; SAWA, YOICHI ET AL: "Aralkylcyclohexanemethylamines" XP002353759 gefunden im STN Database accession no. 88:22336 & JP 52 083818 A2 (TAKEDA CHEMICAL INDUSTRIES, LTD., JAPAN) 13. Juli 1977 (1977-07-13)**
- **SORBERA ET AL.: "Duloxetine Oxalate" DRUGS OF THE FUTURE, Bd. 25, Nr. 9, 2000, Seiten 907-916, XP002350273 in der Anmeldung erwähnt**
- **BERMAN ET AL.: "Monoamine Depletion in Unmedicated Depressed Subjects" BIOLOGICAL PSYCHIATRY, Bd. 51, 2002, Seiten 469-473, XP002350274 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 768 948 B1

**Beschreibung**

Die Erfindung betrifft substituierte Aminoverbindungen, Verfahren zu deren Herstellung, Arzneimittel enthaltend diese Verbindungen sowie die Verwendung dieser Stoffe zur Herstellung von Arzneimitteln, vorzugsweise zur Behandlung von Depressionen, Angstzuständen, Schmerzen und Harninkontinenz, und Verfahren zur Behandlung dieser Erkrankungen.

Die Monoaminwiederaufnahmehemmer aus der Klasse der Trizyklischen Antidepressiva (TCAs) werden seit den 60iger Jahren erfolgreich zur Behandlung von Depressionen eingesetzt. Die Relevanz von Dysfunktionen der monoaminergen Systeme bei psychiatrischen Erkrankungen findet aufgrund der präklinisch und klinisch belegten antidepressiven Wirkungen von TCAs, selektiven Serotoninwiederaufnahmehemmern (sog. SSRIs), selektiven Noradrenalinwiederaufnahmehemmern, gemischten Serotonin- und Noradrenalinwiederaufnahmehemmern (sog. SNRIs), Monoaminoxidase-Hemmern sowie Modulatoren diverser Serotonin- und Noradrenalinrezeptorsubtypen breite Anerkennung (Berman et al., Biol Psychiatry, 2002 Mar 15;51 (6):469-73). Darüber hinaus sind Antidepressiva wichtige Adjuvantien in der Schmerztherapie, insbesondere bei chronischen Schmerzen. Aber auch eine eigenständige analgetische Wirkung wird von Monoaminwiederaufnahmehemmern induziert, indem sie die absteigende Hemmung spinaler nocicpetiver Signale aktivieren. Auch sind gute Erfolge bei der Behandlung von Harninkontinenz durch Einsatz von Monoaminwiederaufnahmehemmern beschrieben (Sorbera et al., Drugs of the future, 2000, Vol 25, Seite 907-916). Monoaminwiedeaufnahmehemmer sind außerdem zur Behandlung von Angstzuständen, Fibromyalgia, Essstörungen, Bulimie, Hyperaktivität (Attention deficit hyperactivity disorder; ADHD), Drogenabhängigkeit, -sucht und -entzug, Trichotillomanie, Hauterkrankungen wie Postherpetische Neuralgie und Pruritus, Gedächtnisstörungen, kognitiven Störungen und Morbus Alzheimer geeignet.

Die therapeutische Verwendung der bisher zugelassenen Antidepressiva ist limitiert durch die häufig auftretenden unerwünschten Nebenwirkungen. Insbesondere sind hier Konstipation, Harnretention, Mundtrockenheit, Akkomodationsstörungen, orthostatische Hypotension mit Tachykardie, Sedation, Serotoninsyndrom, sexuelle Dysfunktionen, Schwindel, kognitive Dysfunktionen und QT-Verlängerungen incl. Torsade de pointes zu nennen. Bei der Behandlung von psychiatrischen Erkrankungen sind ein später Wirkeintritt, eine hohe Rückfallrate sowie eine fehlende Wirkung bei 20-30 % der Patienten nachteilig.

Die der Erfindung zugrunde liegende Aufgabe ist die Bereitstellung von neuen potenten Monoaminwiederaufnahmehemmern mit therapeutisch relevanten Wirkkomponenten bei Depressionen, Angstzuständen, Schmerzen und Harninkontinenz. Die Wirkungen sollten entscheidend auf einer Wiederaufnahmehemmung von Serotonin (5-HT), Noradrenalin (NA) oder aber einer Kombination dieser Mechanismen basieren. Die Substanzen sollen dabei ein verbessertes Wirkprofil und eine günstigere Verträglichkeit aufweisen.

Es wurde nun gefunden, dass Derivate der nachstehenden allgemeinen Formel I eine deutliche Hemmung der 5-HT und NA-Wiederaufnahme bewirken.

Gegenstand der vorliegenden Erfindung sind daher Aminoverbindungen der allgemeinen Formel **I**,

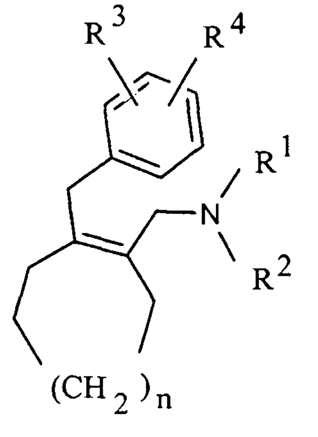

I

in der

$R^1$ für eine geradkettige oder verzweigte $C_{1-4}$-Alkylgruppe,

$R^2$ für Wasserstoff oder den Rest $R^1$,

$R^3$ und $R^4$ unabhängig voneinander für die Gruppe $R^2$, Cl, F, Br, den Rest $OR^2$, $CF_3$, $OCF_3$, $OCH_2F$, $OCHF_2$, zu-

sammengenommen für die Methylendioxygruppe oder eine ankondensierte Benzogruppe, die gegebenenfalls mit der Gruppe $R^2$, Cl, F, Br, dem Rest $OR^2$, $CF_3$, $OCF_3$, $OCH_2F$ oder $OCHF_2$ substituiert ist, stehen

und n = 0, 2 oder 3 bedeutet.

Die erfindungsgemäßen Verbindungen können sowohl in Form der freien Base, als auch in Form von pharmazeutisch annehmbaren Salzen vorliegen.

Bevorzugt sind Verbindungen, in denen $R^1$ für Methyl, $R^2$ für Wasserstoff oder Methyl und $R^3$ und $R^4$ für Wasserstoff oder Cl stehen und n = 0, 2 oder 3 bedeutet.

Besonders bevorzugt sind folgende erfindungsgemäße Verbindungen sowie deren Salze:

[2-(4-Chlorbenzyl)-cyclohept-1-enylmethyl]-dimethylamin und das entsprechende Hydrochlorid **(1)**

[2-(4-Chlorbenzyl)-cyclopent-1-enylmethyl]-dimethylamin und das entsprechende Hydrochlorid **(2a)**

[2-(4-Chlorbenzyl)-cyclooct-1-enylmethyl]-dimethylamin und das entsprechende Hydrochlorid **(2b)**

[2-(4-Chlorbenzyl)-cyclohept-1-enylmethyl]-methylamin und das entsprechende Hydrochlorid **(3).**

Erfindungsgegenstand ist auch ein Verfahren zur Herstellung der substituierten Aminoverbindungen der allgemeinen Formel **I**, das gekennzeichnet ist durch die Umsetzung von tertiären Alkoholen der allgemeinen Formel **II**,

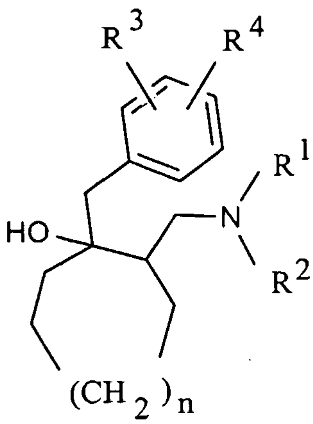

II

wobei $R^1$ bis $R^4$ und n dieselbe Bedeutung wie in Formel **I** haben, mit halbkonzentrierten oder konzentrierten organischen oder anorganischen Säuren, insbesondere Bromwasserstoffsäure in einen Temperaturbereich von 0°C bis 130°C, wobei die tertiären Alkohole der allgemeinen Formel **II** dadurch erhalten werden, dass β-Aminoketone der allgemeinen Formel **III,**

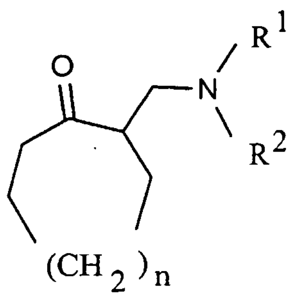

III

wobei $R^1$, $R^2$ und n dieselbe Bedeutung wie in Formel **I** besitzen, mit einer metallorganischen Verbindung der Formel **IV,**

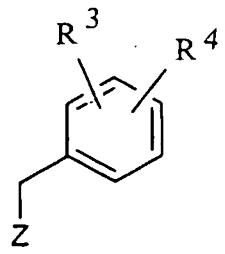

IV

in der Z für MgCl, MgBr, MgI oder Li steht und $R^3$ und $R^4$ wie oben definiert sind, umgesetzt werden.

Die Umsetzung der Verbindungen **III** und **IV** wird in einem aliphatischen Ether, beispielsweise Diethylether und/oder Tetrahydrofuran, bei Temperaturen zwischen -70°C und +60°C durchgeführt. Verbindungen der Formel **IV**, in der Z für ein Lithiumatom steht, werden dabei aus Verbindungen der Formel **IV**, in der Z Br oder I bedeutet, durch Halogen-Lithiumaustausch mittels z.B. einer n-Butyllithium/n-Hexan-Lösung gewonnen.

Stehen $R^3$ und/oder $R^4$ in Verbindungen der allgemeinen Formel I für den Methoxyrest, lassen sich durch Umsetzung mit Diisobutylaluminiumhydrid in einem aromatischen Kohlenwasserstoff wie Toluol oder Xylol bei einer Temperatur zwischen 60°C und 130°C Verbindungen der Formel I herstellen, in denen $R^3$ und/oder $R^4$ für eine Hydroxygruppe stehen.

Man kann solche Verbindungen auch direkt aus Verbindungen der Formel **II**, in denen $R^3$ und/oder $R^4$ für $OCH_3$ stehen, erhalten, indem man Verbindungen der Formel **II** mit einer Lösung von Bromwasserstoff in Eisessig zum Rückfluss erhitzt.

Verbindungen der allgemeinen Formel **I**, in denen $R^2$ für Wasserstoff steht, sind aus entsprechenden Verbindungen der Formel **I** mit $R^2$ = Methyl durch Erhitzen mit Chlorameisensäurephenylester, gefolgt von basischer Hydrolyse, z.B. mit Natronlauge in höher siedenden Alkoholen wie Ethylenglykol, zugänglich.

Die Verbindungen der Formel **I** lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Die erfindungsgemäßen substituierten Aminoverbindungen der allgemeinen Formel **I** sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße substituierte Aminoverbindung der allgemeinen Formel I sowie gegebenenfalls physiologisch verträgliche Hilfsstoffe enthalten. Vorzugsweise eignen sich die erfindungsgemäßen Arzneimittel zur Bekämpfung von Schmerzen (insbesondere chronischer Schmerz, neuropathischer Schmerz, Entzündungsschmerz), Migräne, Fibromyalgia und zur Behandlung oder Prophylaxe von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen), Zyklothyme Persönlichkeitsstörungen (Hypomanie und milde Depressionen)), Angstzuständen (Subtypen Generalisierte Angststörungen, Panikattacken, Zwangssyndromen , social anxiety disorder, Phobien, PSTD), Schlafstörungen, Harninkontinenz (Stress- und Drang-), Essstörungen, Bulimie, Hyperaktivität (Attention deficit hyperactivity disorder; ADHD), Drogenabhängigkeit, -sucht und -entzug, Trichotillomanie, Hauterkrankungen wie Postherpetische Neuralgie und Pruritus, Gedächtnisstörungen; kognitiven Störungen, Psychosen und/oder Morbus Alzheimer.

Die Verwendung wenigstens einer substituierten Aminoverbindung der allgemeinen Formel I zur Herstellung eines Arzneimittels zur Bekämpfung von Schmerzen (insbesondere chronischer Schmerz, neuropathischer Schmerz, Entzündungsschmerz), Migräne, Fibromyalgia und zur Behandlung oder Prophylaxe von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen), Zyklothyme Persönlichkeitsstörungen (Hypomanie und milde Depressionen)), Angstzuständen (Subtypen Generalisierte Angststörungen, Panikattacken, Zwangssyndromen , social anxiety disorder, Phobien, PSTD), Schlafstörungen, Harninkontinenz (Stress- und Drang-), Essstörungen, Bulimie, Hyperaktivität (Attention deficit hyperactivity disorder; ADHD), Drogenabhängigkeit, -sucht und -entzug, Trichotillomanie, Hauterkrankungen wie Postherpetische Neuralgie und Pruritus, Gedächtnisstörungen, kognitiven Störungen, Psychosen und/oder Morbus Alzheimer ist ebenfalls Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern,

Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen substituierten Aminoverbindung der allgemeinen Formel I enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen Verbindung der allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 500 mg/kg, vorzugsweise 0,05 bis 5 mg/kg Körpergewicht des Patienten wenigstens eines erfindungsgemäßen substituierten Aminoverbindungen der allgemeinen Formel **I** appliziert.

Weiter betrifft die Erfindung auch Verfahren zur Behandlung von Depressionen, Schmerzen und Harninkontinenz, bei denen die erfindungsgemäßen Verbindungen verwendet werden.

## Beispiele

Die nachfolgenden Beispiele dienen zur näheren Erläuterung des erfindungsgemäßen Verfahrens.

Als stationäre Phase für die Säulenchromatographie wurde Kieselgel 60 (0,040 - 0,063 mm) der Firma E. Merck, Darmstadt, eingesetzt.

### Beispiel 1

<u>[2-(4-Chlorbenzyl)-cyclohept-1-enylmethyl]-dimethylamin; Hydrochlorid</u>

Stufe 1:

<u>1-(4-Chlorbenzyl)-2-dimethylaminomethyl-cycloheptanol</u>

Zu einer frisch bereiteten Lösung des Grignard-Reagenzes aus 6,17 g Magnesiumspänen und 40,50 g 4-Chlorbenzylchlorid in 500 ml absolutem Diethylether wurde bei 20°C unter Rühren eine Lösung von 32,00 g 2-Dimethylaminomethylcycloheptanon in 190 ml absolutem Diethylether getropft. Nach beendeter Zugabe wurde noch 2 Stunden bei 20°C weitergerührt. Dann wurde unter Eiskühlung durch Zutropfen von 100 ml einer gesättigten Ammoniumchloridlösung, gefolgt von 200 ml destillierten Wassers, zersetzt. Die organische Phase wurde abgetrennt, die wässrige zweimal mit je 200 ml Essigsäureethylester extrahiert. Die vereinigten organischen Extrakte wurden mit gesättigter Natriumchloridlösung gewaschen, über Natriumsulfat getrocknet und im Vakuum eingedampft. Der ölige Rückstand wurde durch Säulenchromatographie mit Essigsäureethylester als Elutionsmittel gereinigt, wobei 39,69 g der Titelverbindung in Form der freien Base anfielen. Diese wurden in 270 ml 2-Butanon gelöst und durch Zugabe von 2,8 ml Wasser und 17,4 ml Trimethylchlorsilan ins Hydrochlorid übergeführt. Dabei fielen 37,30 g (59,4 % der Theorie) der Titelverbindung als Diastereomerengemisch in Form farbloser Kristalle an. Schmelzpunkt: 235 - 237°C

Stufe 2:

[2-(4-Chlorbenyzl)-cyclohept-1-enylmethyl]-dimethylamin; Hydrochlorid

33,23 g des Produkts aus Stufe 1 wurden in 140 ml Bromwasserstoffsäure (47 % HBr) 1 Stunde zum Rückfluss erhitzt. Dann wurde im Vakuum eingedampft und der Rückstand aus Ethanol umkristallisiert. Nach Überführen des Produkts in die freie Base mit wässriger Natriumcarbonatlösung wurden daraus durch Hydrochloridfällung mit Trimethylchlorsilan/Wasser in 2-Butanon wie in Stufe 1 beschrieben 9,35 g (29,8 % der Theorie) der Titelverbindung in Form farbloser Kristalle erhalten. Schmelzpunkt: 220 - 222°C

**Beispiel 2**

Unter Anwendung der in Beispiel 1 beschriebenen Verfahrensweise und bei Einsatz von 2-Dimethylaminomethyl-cyclopentanon bzw. 2-Dimethylaminomethylcyclooctanon anstelle des entsprechenden Cycloheptanonderivats in Stufe 1 wurden analog erhalten:

a) [2-(4-Chlorbenzyl)-cyclopent-1-enylmethyl]-dimethylamin; Hydrochlorid
Schmelzpunkt: 191°C

b) [2-(4-Chlorbenzyl)-cyclooct-1-enylmethyl]-dimethylamin; Hydrochlorid
Schmelzpunkt: 234 - 237°C

**Beispiel 3**

[2-(4-Chlorbenzyl)-cyclohept-1-enylmethyl]-methylamin; Hydrochlorid

0,19 g des Produkts aus Beispiel 1 als freie Base wurden mit 0,1 ml Chlorameisensäurephenylester in 12 ml wasserfreiem Toluol 2 Stunden zum Rückfluss erhitzt. Nach dem Abkühlen wurde nacheinander mit 2,5 N Natronlauge, Wasser, 1 N Salzsäure bzw. einer gesättigten Nariumchloridlösung gewaschen und über Natriumsulfat getrocknet. Der durch Eindampfen im Vakuum erhaltenen Rückstand wurde in 6 ml Ethylenglykol aufgenommen und mit 1,4 ml 5N Natronlauge 4,5 Stunden auf 110°C erhitzt. Nach dem Abkühlen wurde mit 20 ml Wasser verdünnt und dreimal mit je 10 ml Dichlormethan extrahiert. Die Extrakte wurden mit gesättigter Natriumchloridlösung gewaschen und über Natriumsulfat getrocknet. Der durch Eindampfen im Vakuum erhaltene ölige Rückstand wurde wie in Beispiel 1, Stufe 1, beschrieben mit Trimethylchlorsilan/Wasser in 2-Butanon ins Hydrochlorid übergeführt. Dabei fielen 0,08 g (39 % der Theorie) der Titelverbindung in Form nahezu farbloser Kristalle an.
$^1$H-NMR (DMSO-$d_6$); 1,12 - 1,70 (m, 6H); 2,04 - 2,13 (m, 2H); 2,30 - 2,38 (m, 2H); 2,71 (s, 3H); 3,53 (s, 2H); 3,82 - 3,90 (m, 2H); 7,13 - 7,40 (m, 4H)

**Pharmakologische Untersuchungen**

**a) Untersuchungen der 5-HT- und NA-Wiederaufnahmehemmung**

Um diese in vitro Studien durchführen zu können, werden Synaptosomen aus Rattenhirnarealen frisch isoliert. Es findet jeweils eine sogenannte "$P_2$"-Fraktion Verwendung, die exakt nach der Vorschrift von Gray, E. G. und Whittaker, V. P. (J. Anat. 76, 79-88, 1962) präpariert wird. Für die NA- Wiederaufnahme werden diese vesikulären Partikel aus dem Hypothalamus, für die 5-HT- Wiederaufnahme aus der Medulla + Pons-Region von männlichen Rattengehirnen isoliert.
Folgende Kenndaten wurden für die NA- und 5-HT- Wiederaufnahme ermittelt:

NA-Uptake : Km = 0,32 $\pm$ 0,11 $\mu$M

5-HT-Uptake : Km = 0,084 $\pm$ 0,011 $\mu$M

(Jeweils N = 4, d.h. Mittelwerte $\pm$ SEM aus 4 unabhängigen Versuchsreihen, die in Dreifach-Parallelversuchen durchgeführt wurden).
Eine detaillierte Methodenbeschreibung ist in der Publikation von Frink, M., Hennies, H. H., Englberger, W. et al. (Arzneim.-Forsch./Drug Res. 46 (III), 11, 1029-1036, 1996) enthalten (der Ansatz kann auch auf Mikrotiterplatten (250 $\mu$l / well) bei Zimmertemperatur durchgeführt werden).

Auswertungen:

Neben % Hemmungen bei fixen Testsubstanzkonzentrationen (z.B. 1 x $10^{-6}$ M oder 1 x $10^{-5}$ M im Ansatz), wurden Dosisabhängigkeiten überprüft. Hierbei werden $IC_{50}$-Werte erhalten, die gemäß der "Cheng-Prusoff Gleichung" (Cheng, Y. C. und Prusoff, W. H., Biochem. Pharmacol. 22, 3099-3108, 1973) in Inhibitorkonstanten ($K_i$) umgerechnet werden können. Die $IC_{50}$ Werte wurden mit Hilfe des Computer-Programms "Figure P" (Version 6.0, Biosoft, Cambridge, England) erhalten. Km-Werte wurden gemäß Lineweaver, H. und Burk, D. (J. Am. Chem. Soc. 56, 658-666, 1934) berechnet. Um $K_D$-Werte darzustellen, ist das Computer-Programm "Ligand" (Version 4, Biosoft, England) angewendet worden.

Für die Verbindungen der Beispiele 1 und 2b wurden eine dosisabhängige Hemmungen der 5-HT und NA-Wiederaufnahme bestimmt. Die entsprechenden Ergebnisse sind in der nachfolgenden Tabelle zusammengefasst.

Tabelle 1

| **Verbindung gemäß Beispiel Nr.** | **5-HT-Wiederaufnahmeinhibition; Ki (μmol/l)** | **NA-Wiederaufnahmeinhibition; Ki (μmol/l)** |
|---|---|---|
| Beispiel 1 | 0,003 | 0,03 |
| Beispiel 2b | 0,03 | 0,34 |

**b) Untersuchungen der analgetischen Wirkungen im Formalin-Test an der Maus**

Der Formalin Test (Dubuisson, D. and Dennis, S.G., 1977, Pain, 4, 161 - 174) stellt ein Modell für den akuten sowie den chronischen Schmerz dar. In den hier vorgestellten Untersuchungen wurde die chronische Schmerzkomponente ausgewertet.

Durch eine einmalige Formalin-Injektion in die dorsale Seite einer Hinterpfote wird bei freibeweglichen Versuchstieren eine biphasige nociceptive Reaktion induziert, die durch Beobachtung von drei deutlich voneinander unterscheidbaren Verhaltensmustern erfasst wird.

Formalin wird mit dem Volumen von 20 μl und einer Konzentration von 1 % subkutan in die dorsale Seite der rechten Hinterpfote jedes Tieres appliziert. Die vom Normalverhalten (Score 0) differierenden spezifischen Verhaltensänderungen, wie Anheben (Score 1) und Schütteln der Pfote (Score 2), sowie Beiss- und Leckreaktionen (Score 3) werden bis 60 min nach Formalinapplikation in Teilintervallen von 3 min kontinuierlich beobachtet und registriert. Die Verhaltensänderungen werden unterschiedlich gewichtet (Score 0-3) und eine Pain-Rate (PR) errechnet mit folgender Formel:

$$PR = [(T_0 \times 0) + (T_1 \times 1) + (T_2 \times 2) + (T_3 \times 3) / 180.$$

Dabei entsprechen $T_0$, $T_1$, $T_2$, $T_3$ jeweils der Zeit in Sekunden, in der das Tier die Verhaltensweisen 0, 1, 2, oder 3 zeigte. Die Gruppengröße beträgt 10 Tiere (n=10). Basierend auf den PR-Berechnungen wurde die Substanzwirkung als Änderung gegen eine Kontrolle in Prozent ermittelt. Die $ED_{50}$-Bestimmung erfolgte mittels Regressionsanalyse.

Für die Verbindung gemäß Beispiel 1 wurde eine dosisabhängige Hemmung des nociceptiven Verhaltens beobachtet. Das Ergebnis ist in der nachfolgenden Tabelle dargestellt.

Tabelle 2

| **Verbindung gemäß Beispiel Nr.** | **Applikationsart** | **$ED_{50}$-Wert, mg/kg** |
|---|---|---|
| Beispiel 1 | i. v. | 4,71 |

**c) Untersuchungen der analgetischen Wirkungen im Writhing-Test an der Maus**

Der Writhing-Test an der Maus stellt ein Modell für akuten Schmerz dar. Die hier zusammengefassten Experimente wurden nach einer Methode, modifiziert nach Hendershot LC, Forsaith J, J Pharmacol Exp Ther 125:237-240 (1959), durchgeführt. Die Tiere erhalten als Schmerzstimulus 0.3 ml/Tier einer 0.02%igen wässrigen Phenylchinonlösung unter Zusatz von 5% Ethanol intraperitoneal appliziert. Diese wird 30 min (Testsubstanz p.o.) oder 10 min (Testsubstanz i.v.) nach der Gabe der Testsubstanz appliziert. Registriert werden die Schmerz-induzierten Streckbewegungen (sog. Writhingreaktionen = Durchdrücken des Körpers mit Abstrecken der Hinterextremitäten) 5-20 min nach der Phenylchinon-Gabe. Verglichen werden die substanzbehandelten Tiere mit einer Kontrollgruppe, die physiologische Kochsalzlösung

erhielt. Gruppengröße n=10

Für die Verbindung gemäß Beispiel 1 wurde eine dosisabhängige Hemmung des nociceptiven Verhaltens beobachtet. Das Ergebnis ist in der nachfolgenden Tabelle dargestellt.

Tabelle 3

| Verbindung gemäß Beispiel Nr. | Applikationsart | $ED_{50}$-Wert, mg/kg |
|---|---|---|
| Beispiel 1 | i.v. | 3,04 |

**d) Untersuchung der antidepressiven Wirkung im Forced swimming-Test (Porsolt-Test) an der Maus**

Die Untersuchungen zur Bestimmung der antidepressiven Wirkung der erfindungsgemäßen Verbindungen der Formel I wurden im Forced swimming -Test (Porsolt-Test) an der Maus durchgeführt (Porsolt, R. et al., Arch. Int. Pharmacodyn. Vol. 229, S. 327-336, (1977)). Männliche Mäuse (20-25 g Körpergewicht) wurden individuell für eine Dauer von 6 Minuten in ein flaches Wasserbecken gesetzt, aus dem sie nicht entfliehen konnten und somit gezwungen waren zu schwimmen. Nach einiger Zeit gaben die Tiere ihre Schwimmversuche auf und gingen in eine Immobilitätsphase über. In dem Intervall von 2-6 Minuten nach dem Einbringen des Tieres wurde Dauer der Immobilitätsphase bestimmt. Substanz- und Vehikelgruppen umfassen jeweils 10 Tiere. Änderungen der Dauer der Immobilitätsphase werden relativ zur Vehikelkontrolle angegeben.

Antidepressiva induzieren eine Verkürzung der Immobilitätsphase.

Für die Verbindung gemäß Beispiel Nr. 1 wurde eine signifikante Verkürzung der Immobilitätsphase und somit eine antidepressive Wirkung im Forced swimming-Test ermittelt. Die Ergebnisse sind in der nachfolgenden Tabelle dargestellt.

Tabelle 4

| Verbindung gemäß Beispiel Nr. | Applikationsart | Dosierung mg/kg | Relative Dauer der Immobilitätsphase versus Kontrolle |
|---|---|---|---|
| Beispiel 1 | i.p. | 10 | + 9 % |
| Beispiel 1 | i.p. | 21,5 | - 34 % |
| Beispiel 1 | i.p. | 31,6 | -67 % *** |
| Beispiel 1 | i.p. | 46,4 | -76 % *** |
| Student's T-Test; ***: $p < 0,001$ | | | |

**Patentansprüche**

**1.** Substituierte Aminoverbindungen der allgemeinen Formel I,

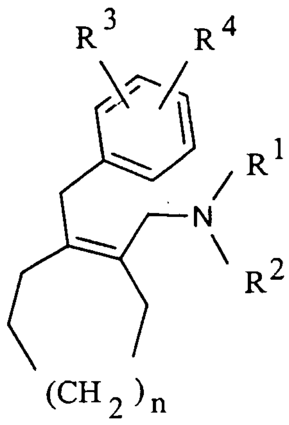

I

I

worin

$R_1$ für eine geradkettige oder verzweigte $C_{1-4}$-Alkylguppe,
$R_2$ für Wasserstoff oder den Rest $R^1$,
$R^3$ und $R^4$ unabhängig voneinander für die Gruppe $R^2$, Cl, F, Br, den Rest $OR^2$, $CF_3$, $OCF_3$, $OCH_2F$, $OCHF_2$, zusammengenommen für die Methylendioxygruppe oder eine ankondensierte Benzogruppe, die gegebenenfalls mit der Gruppe $R^2$, Cl, F, Br, dem Rest $OR^2$, $CF_3$, $OCF_3$, $OCH_2F$ oder $OCHF_2$ substituiert ist, stehen
und n = 0, 2 oder 3 bedeutet,

in Form ihrer freien Basen als auch in Form von pharmazeutisch annehmbaren Salzen.

**2.** Verbindungen gemäß Anspruch 1, **dadurch gekennzeichnet, dass** $R^1$ für Methyl, $R^2$ für Wasserstoff oder Methyl und $R^3$ und $R^4$ für Wasserstoff oder Cl stehen und n =0,2 oder 3 bedeutet.

**3.** Eine substituierte Aminoverbindung gemäß Anspruch 1 aus der Gruppe umfassend:

[2-(4-Chlorbenzyl)-cyclohept-1-enylmethyl]-dimethylamin und das entsprechende Hydrochlorid **(1)**
[2-(4-Chlorbenzyl)-cyclopent-1-enylmethyl]-dimethylamin und das entsprechende Hydrochlorid **(2a)**
[2-(4-Chlorbenzyl)-cyclooct-1-enylmethyl]-dimethylamin und das entsprechende Hydrochlorid **(2b)**
[2-(4-Chlorbenzyl)-cyclohept-1-enylmethyl]-methylamin und das entsprechende Hydrochlorid **(3).**

**4.** Verfahren zur Herstellung von substituierten Aminoverbindungen der allgemeinen Formel **I** gemäß einem der Ansprüche 1 bis 3, **dadurch gekennzeichnet, dass** tertiäre Alkohole der allgemeinen Formel **II**,

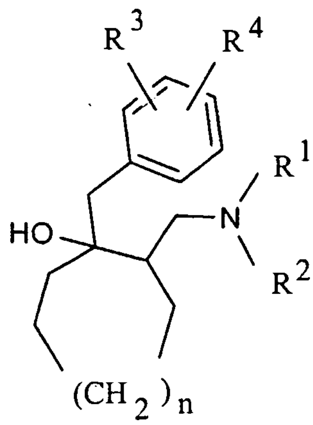

II

worin $R^1$ bis $R^4$ und n dieselbe Bedeutung wie in Formel I haben, mit halbkonzentrierten oder konzentrierten organischen oder anorganischen Säuren in einem Temperaturbereich von 0°C bis 130°C umgesetzt werden,
wobei man Verbindungen der Formel **I**, in der $R^3$ und/oder $R^4$ für die Hydroxygruppe stehen, herstellt, indem man entweder Verbindungen der Formel **I,** in der $R^3$ und/oder $R^4$ für den Methoxyrest stehen, mit Diisobutylaluminiumhydrid in einem aromatischen Kohlenwasserstoff bei 60°C bis 130°C umsetzt
oder
Verbindungen der Formel **II**, in der $R^3$ und/oder $R^4$ für den Methoxyrest stehen, mit einer Lösung von Bromwasserstoff in Eisessig zum Rückfluss erhitzt,
und
Verbindungen der allgemeinen Formel **I**, in der $R^2$ für Wasserstoff steht, aus entsprechenden Verbindungen der Formel **I** mit $R^2$ gleich Methyl durch Erhitzen mit Chlorameisensäurephenylester, gefolgt von basischer Hydrolyse, herstellt,
und wobei die tertiären Alkohole der allgemeinen Formel **II** hergestellt werden, indem β-Aminoketone der allgemeinen Formel **III**,

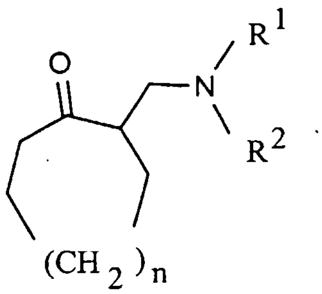

III

worin $R^1$, $R^2$ und n dieselbe Bedeutung wie in Formel besitzen, mit einer metallorganischen Verbindung der Formel **IV**,

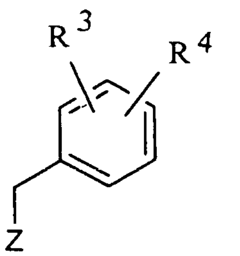

IV

in der Z für MgCl, MgBr, MgI oder Li steht und $R^3$ und $R^4$ wie in Formel I definiert sind, umgesetzt werden.

**5.** Verfahren nach Anspruch 4, **dadurch gekennzeichnet, dass** als Säure Bromwasserstoffsäure eingesetzt wird.

**6.** Verfahren nach Anspruch 4, **dadurch gekennzeichnet, dass** die Umsetzung der Verbindungen **III** und **IV** in einem aliphatischen Ether bei Temperaturen zwischen -70°C und +60°C erfolgt, wobei Verbindungen der Formel **IV,** in der Z für ein Lithiumatom steht, aus Verbindungen der Formel **IV,** in der Z Br oder I bedeutet, durch Halogen-Lithiumaustausch hergestellt werden.

**7.** Arzneimittel enthaltend als Wirkstoff wenigstens eine Verbindung der allgemeinen Formel **I** gemäß einem der Ansprüche 1 bis 3 und gegebenenfalls physiologisch verträgliche Hilfsstoffe.

**8.** Arzneimittel gemäß Anspruch 7 zur Bekämpfung von Schmerzen.

**9.** Arzneimittel gemäß Anspruch 7 zur Behandlung oder Prophylaxe von Depressionen.

**10.** Arzneimittel gemäß Anspruch 7 zur Behandlung oder Prophylaxe von Angstzuständen.

**11.** Arzneimittel gemäß Anspruch 7 zur Behandlung oder Prophylaxe von Harninkontinenz.

**12.** Verwendung wenigstens einer Verbindung der allgemeinen Formel **I** gemäß einem der Ansprüche 1 bis 3 zur Herstellung eines Arzneimittels zur Bekämpfung von Schmerzen.

**13.** Verwendung wenigstens einer Verbindung der allgemeinen Formel **I** gemäß einem der Ansprüche 1 bis 3 zur Herstellung eines Arzneimittels zur Behandlung oder Prophylaxe von Depressionen, Angstzuständen, Harninkontinenz, Fibromyalgia, Essstörungen, Bulimie, Hyperaktivität (Attention deficit hyperaactivity disorder; ADHD), Drogenabhängigkeit, -sucht und -entzug, Trichotillomanie, Postherpethischer Neuralgie, Pruritus, Gedächtnisstörungen, kognitiven Störungen und/oder Morbus Alzheimer.

**Claims**

**1.** Substituted amino compounds of the general formula I

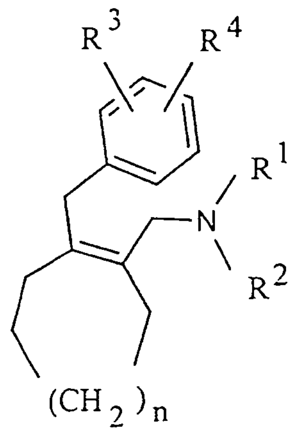

I

wherein

$R^1$ represents a straight-chained or branched $C_{1-4}$-alkyl group,
$R^2$ represents hydrogen or the radical $R^1$,
$R^3$ and $R^4$, independently of one another, represent the group $R^2$, Cl, F, Br, the radical $OR^2$, $CF_3$, $OCF_3$, $OCH_2F$, $OCHF_2$, or
$R^3$ and $R^4$ together represent a methylenedioxy group or a fused benzo group which is optionally substituted by the group $R^2$, Cl, F, Br, the radical $OR^2$, $CF_3$, $OCF_3$, $OCH_2F$ or $OCHF_2$,
and n = 0, 2 or 3,

in the form of their free bases and in the form of pharmaceutically acceptable salts.

**2.** Compounds according to claim 1, **characterised in that** $R^1$ represents methyl, $R^2$ represents hydrogen or methyl and $R^3$ and $R^4$ represent hydrogen or Cl, and n = 0, 2 or 3.

**3.** A substituted amino compound according to claim 1 from the group comprising:

[2-(4-chlorobenzyl)-cyclohept-1-enylmethyl]-dimethylamine and the corresponding hydrochloride **(1)**
[2-(4-chlorobenzyl)-cyclopent-1-enylmethyl]-dimethylamine and the corresponding hydrochloride **(2a)**
[2-(4-chlorobenzyl)-cyclooct-1-enylmethyl]-dimethylamine and the corresponding hydrochloride **(2b)**
[2-(4-chlorobenzyl)-cyclohept-1-enylmethyl]-methylamine and the corresponding hydrochloride **(3).**

**4.** Process for the preparation of substituted amino compounds of the general formula I according to any one of claims 1 to 3, **characterised in that** tertiary alcohols of the general formula II

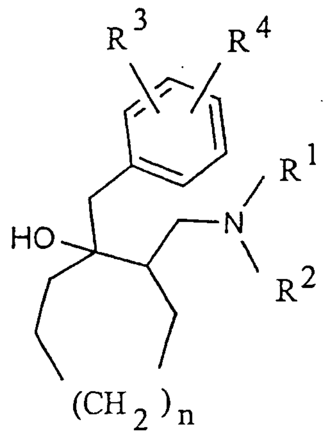

II

wherein $R^1$ to $R^4$ and n have the same meaning as in formula I, are reacted with semi-concentrated or concentrated organic or inorganic acids in a temperature range of from 0°C to 130°C,
wherein compounds of formula **I** in which $R^3$ and/or $R^4$ represent(s) the hydroxy group are prepared either by reacting compounds of formula I in which $R^3$ and/or $R^4$ represent(s) a methoxy radical with diisobutylaluminium hydride in an aromatic hydrocarbon at from 60°C to 130°C
or
by heating compounds of formula **II** in which $R^3$ and/or $R^4$ represent(s) a methoxy radical at reflux with a solution of hydrogen bromide in glacial acetic acid,
and
compounds of the general formula I in which $R^2$ represents hydrogen are prepared from corresponding compounds of formula I wherein $R^2$ is methyl by heating with chloroformic acid phenyl ester followed by basic hydrolysis,
and wherein the tertiary alcohols of the general formula **II** are prepared by reacting β-amino ketones of the general formula **III**

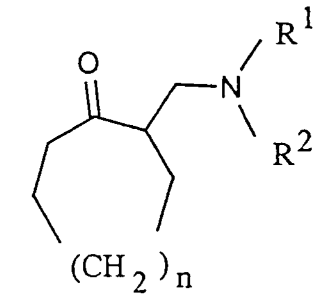

III

wherein $R^1$, $R^2$ and n have the same meaning as in formula **I**, with an organometallic compound of formula **IV**

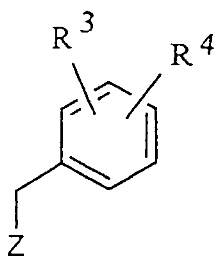

IV

in which Z represents MgCl, MgBr, MgI or Li and $R^3$ and $R^4$ are as defined in formula I.

5. Process according to claim 4, **characterised in that** hydrobromic acid is used as the acid.

6. Process according to claim 4, **characterised in that** the reaction of compounds **III** and **IV** is carried out in an aliphatic ether at temperatures of from -70°C to +60°C, compounds of formula **IV** in which Z represents a lithium atom being obtained from compounds of formula **IV** in which Z represents Br or I by halogen-lithium exchange.

7. Medicament comprising at least one compound of the general formula **I** according to any one of claims 1 to 3 as active ingredient and, optionally, physiologically acceptable auxiliary substances.

8. Medicament according to claim 7 for the control of pain.

9. Medicament according to claim 7 for the treatment or prophylaxis of depression.

10. Medicament according to claim 7 for the treatment or prophylaxis of anxiety.

11. Medicament according to claim 7 for the treatment or prophylaxis of urinary incontinence.

12. Use of at least one compound of the general formula **I** according to any one of claims 1 to 3 in the preparation of a medicament for the control of pain.

13. Use of at least one compound of the general formula **I** according to any one of claims 1 to 3 in the preparation of a medicament for the treatment or prophylaxis of depression, anxiety, urinary incontinence, fibromyalgia, eating disorders, bulimia, hyperactivity (attention deficit hyperactivity disorder; ADHD), drug dependency, addiction and withdrawal, trichotillomania, post-herpetic neuralgia, pruritus, memory disorders, cognitive disorders and/or Alzheimer's disease.

**Revendications**

1. Composés amino-substitués de formule générale I,

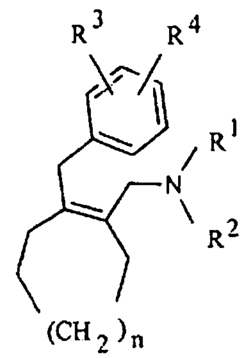

I

dans laquelle

$R^1$ représente un groupe alkyle en $C_1$ à $C_4$ linéaire ou ramifié,
$R^2$ représente l'hydrogène ou le reste $R^1$,
$R^3$ et $R^4$ représentent, indépendamment l'un de l'autre, le groupe $R^2$, Cl, F, Br, le reste $OR^2$, $CF_3$, $OCF_3$, $OCH_2F$, $OCHF_2$, conjointement le groupe méthylènedioxy ou un groupe benzocondensé, qui est éventuellement substitué avec le groupe $R^2$, Cl, F, Br, le reste $OR^2$, $CF_3$, $OCF_3$, $OCH_2F$ ou $OCHF_2$,
et
n a la valeur 0, 2 ou 3,

sous forme de leurs bases libres ainsi que sous forme de sels pharmaceutiquement acceptables.

2. Composés suivant la revendication 1, **caractérisés en ce que** $R^1$ représente un reste méthyle, $R^2$ représente

l'hydrogène ou un reste méthyle, et $R^3$ et $R^4$ représentent l'hydrogène ou Cl, et n a la valeur 0, 2 ou 3.

**3.** Composé amino-substitué suivant la revendication 1, du groupe comprenant :

la [2-(4-chlorobenzyl)-cyclohept-1-énylméthyl]-diméthylamine et le chlorhydrate **(1)** correspondant,
la [2-(4-chlorobenzyl)-cyclopent-1-énylméthyl]-diméthylamine et le chlorhydrate **(2a)** correspondant,
la [2-(4-chlorobenzyl)-cyclo-oct-1-énylméthyl]-diméthylamine et le chlorhydrate **(2b)** correspondant,
la [2-(4-chlorobenzyl)-cyclohept-1-énylméthyl]-méthylamine et le chlorhydrate **(3)** correspondant.

**4.** Procédé de production de composés amino-substitués de formule générale I, suivant l'une des revendications 1 à 3, **caractérisé en ce que** des alcools tertiaires de formule générale II,

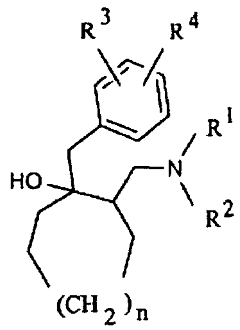

II

dans laquelle $R^1$ à $R^4$ et n ont la même définition que dans la formule I, sont amenés à réagir avec des acides organiques ou inorganiques à demi concentrés ou concentrés, dans une plage de température de 0°C à 130°C, les composés de formule I dans laquelle $R^3$ et/ou $R^4$ représentent le groupe hydroxy étant préparés par réaction à une température de 60°C à 130°C de composés de formule I dans laquelle $R^3$ et/ou $R^4$ représentent le reste méthoxy, avec l'hydrure de diisobutylaluminium dans un hydrocarbure aromatique,
ou bien des composés de formule II dans laquelle $R^3$ et/ou $R^4$ représentent le reste méthoxy étant chauffés au reflux avec une solution de bromure d'hydrogène dans l'acide acétique cristallisable,
et
des composés de formule générale I dans laquelle $R^2$ représente l'hydrogène étant préparés à partir de composés correspondants de formule I dans laquelle $R^2$ est un reste méthyle, par chauffage avec l'ester de phényle de l'acide chloroformique, suivi d'une hydrolyse basique,
et les alcools tertiaires de formule générale II étant préparés par réaction de β-aminocétones de formule générale III,

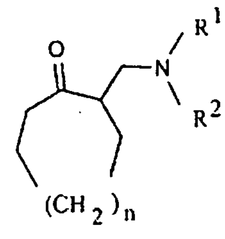

III

dans laquelle $R^1$, $R^2$ et n ont la même définition que dans la formule I, avec un composé organométallique de formule IV,

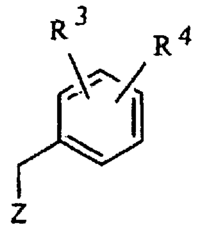

IV

dans laquelle Z représente MgCl, MgBr, MgI ou Li et $R^3$ et $R^4$ sont tels que définis dans la formule I.

**5.** Procédé suivant la revendication 4, **caractérisé en ce que** l'acide utilisé est l'acide bromhydrique.

**6.** Procédé suivant la revendication 4, **caractérisé en ce que** la réaction des composés III et IV est conduite dans un éther aliphatique à des températures comprises entre - 70°C et + 60°C, les composés de formule IV, dans laquelle Z représente un atome de lithium étant préparés à partir de composés de formule IV dans laquelle Z représente Br ou I, par échange halogène-lithium.

**7.** Médicament contenant comme substance active au moins un composé de formule générale I suivant l'une des revendications 1 à 3 et, le cas échéant, des substances auxiliaires physiologiquement acceptables.

**8.** Médicament suivant la revendication 7, destiné à combattre des maladies.

**9.** Médicament suivant la revendication 7, destiné au traitement ou à la prophylaxie de dépressions.

**10.** Médicament suivant la revendication 7, destiné au traitement ou à la prophylaxie d'états d'anxiété.

**11.** Médicament suivant la revendication 7, destiné au traitement ou à la prophylaxie de l'incontinence urinaire.

**12.** Utilisation d'au moins un composé de formule générale I suivant l'une des revendications 1 à 3 pour la préparation d'un médicament destiné au traitement de douleurs.

**13.** Utilisation d'au moins un composé de formule générale I suivant l'une des revendications 1 à 3 pour la préparation d'un médicament destiné au traitement ou à la prophylaxie de dépressions, d'états d'anxiété, d'incontinence urinaire, de fibromyalgie, de troubles de l'alimentation, de boulimie, d'hyperactivité (Attention deficit hyperactivity disorder ; ADHD), de toxicomanie, de dépendance aux drogues et de privation de drogues, de trichotillomanie, d'algies postzostériennes, de prurit, de troubles de la mémoire, de troubles cognitifs et/ou de la maladie d'Alzheimer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **BERMAN et al.** *Biol Psychiatry,* 15. Marz 2002, vol. 51 (6), 469-73
- **SORBERA.** *Drugs of the future,* 2000, vol. 25, 907-916
- Remington's Pharmaceutical Sciences. Mack Publishing Company, 1985, 17
- **VORSCHRIFT VON GRAY, E. G. ; WHITTAKER, V. P.** *J. Anat.,* 1962, vol. 76, 79-88
- **FRINK, M. ; HENNIES, H. H. ; ENGLBERGER, W. et al.** *Arzneim.-Forsch./Drug Res.,* 1996, vol. 46 (11), 1029-1036
- **CHENG, Y. C. ; PRUSOFF, W. H.** *Biochem. Pharmacol,* 1973, vol. 22, 3099-3108
- **LINEWEAVER, H. ; BURK, D.** *J. Am. Chem. Soc.,* 1934, vol. 56, 658-666
- **DUBUISSON, D. ; DENNIS, S.G.** *Pain,* 1977, vol. 4, 161-174
- **HENDERSHOT LC ; FORSAITH J.** *J Pharmacol Exp Ther,* 1959, vol. 125, 237-240
- **PORSOLT, R. et al.** *Arch. Int. Pharmacodyn.,* 1977, vol. 229, 327-336